# EUROPEAN PATENT SPECIFICATION

(11) **EP 2 453 941 B1**
(45) Date of publication and mention of the grant of the patent: **14.12.2016**
(21) Application number: 10732830.4
(22) Date of filing: 13.07.2010
(51) Int. Cl.: A61L 31/14, A61F 2/89, A61F 2/07, A61L 31/08

(54) **COATED MEDICAL DEVICES AND METHODS**
BESCHICHTETE MEDIZINISCHE VORRICHTUNGEN UND VERFAHREN
DISPOSITIFS MÉDICAUX REVÊTUS ET PROCÉDÉS ASSOCIÉS

(30) Priority: 13.07.2009 US 225010 P
(43) Date of publication of application: 23.05.2012
(73) Proprietor: Cook Medical Technologies LLC, Bloomington, IN 47404 (US)
(72) Inventor: GREWE, David, West Lafayette IN 47906 (US); HASELBY, Kenneth, Battleground IN 47920 (US); MILNER, Keith, West Lafayette IN 47906 (US)
(74) Representative: Powell, Stephen David
(86) International application number: PCT/US2010/041833
(87) International publication number: WO 2011/008764

(56) References cited:
- EP-A1- 1 466 570
- WO-A1-2010/030508
- WO-A2-2009/058705
- US-A- 4 143 423

## Description

### Background Art

EP 1 466 570 A1 discloses a modified delivery device for coated medical devices.

Various implantable medical devices are advantageously inserted within body vessels to treat various medical conditions. Minimally invasive techniques and instruments for placement of intraluminal medical devices, such as stents or stent-grafts, have been developed to treat and repair undesirable conditions within body vessels, including treatment of conditions that affect fluid flow within a body vessel.

One or more intraluminal medical devices can be introduced to a point of treatment within a body vessel using a delivery catheter device passed through the vasculature communicating between a remote introductory location and the implantation site, and released from the delivery catheter device at the point of treatment within the body vessel. Radially expandable stents or stent-grafts are typically radially compressed to a low-profile configuration and inserted into a delivery system. These medical devices may be configured for expansion within a body vessel by balloon expansion or self-expansion. Friction between a graft material and a compression device may result in a failure of the device to compress to a desired radial profile and in turn result in excessive friction between graft material and delivery device. Friction may compromise the mechanical integrity of the graft or reduce retention of any therapeutic agents that may be present within the device, compromising the therapeutic effectiveness of the device.

Intraluminal medical devices, such as stent-grafts, typically include a radially-expandable support frame attached to a graft material. Various materials have been used as the graft material, including the biocompatible polyurethane polymer materials. One example of biocompatible polyurethane includes THORALON (THORATEC, Pleasanton, Calif.), described in U.S. Pat. Application Pub. No. 2002/0065552 A1 and U.S. Pat. No. 4,675,361. The biocompatible polyurethane material sold under the trade name THORALON is a polyurethane base polymer (referred to as BPS-215) blended with a siloxane containing surface modifying additive (referred to as SMA-300).

Biocompatible polyurethane polymers have been used in certain vascular applications and are characterized by thromboresistance, high tensile strength, low water absorption, low surface energy, and good flex life. For example, the biocompatible polyurethane material sold under the tradename THORALON is believed to be biostable and to be useful *in vivo* in long term blood contacting applications requiring biostability and leak resistance. Because of its flexibility, THORALON is useful in larger vessels, such as the abdominal aorta, where elasticity and compliance is beneficial.

However, THORALON and other polyurethane polymer materials, in addition to silicone rubber polymers, exhibit high amount of adhesiveness or friction toward other materials. This is especially evidenced when these polymers are placed in high pressure contact with another smooth surface such as nylon, PTFE, metal, glass, etc., and then an attempt is made to slide one of these materials with respect to the other.

### Disclosure of The Invention

According to a first aspect of the present invention, there is provided a medical device system comprising: a sheath having an abluminal surface and a luminal surface; an expandable medical device disposed at least partially within the sheath, the device having an abluminal surface at least partially in contact with the luminal surface of the sheath, and a luminal surface defining a lumen: and a powder coating of one or more of sodium bicarbonate, sodium maleate, sodium gluconate, sodium fumarate, sodium chloride, magnesium bicarbonate and potassium bicarbonate, disposed on at least one of the abluminal surface of the device and the luminal surface of the sheath, wherein the device and the sheath prior to the application of the coating have at least one of a first property of adhesiveness and a first property of friction when in contact with each other, and subsequent to the application of the coating have at least one of a second property of adhesiveness less than the first property of adhesiveness and a second property of friction less than the first property of friction when in contact with each other.

In one embodiment, the invention relates to a medical device system that includes a delivery instrument comprising a sheath having an abluminal surface and a luminal surface, a radially-expandable frame disposed at least partially within the sheath, the frame having an abluminal surface at least partially in contact with the luminal surface of the sheath, and a luminal surface defining a substantially cylindrical lumen, and a fine powder coating, selected from the group consisting of sodium bicarbonate, sodium maleate, sodium gluconate, and sodium fumarate, disposed on at least one of the abluminal surface of the frame and the luminal surface of the sheath. The coefficient of friction between the frame and the sheath subsequent to the application of the coating is less than the coefficient of friction between the frame and the sheath prior to the application of the coating, and in the range of from about 0.2 to about 0.5. The frame and the sheath prior to the application of the coating have at least one of a first property of adhesiveness and a first property of friction when in contact with each other, and subsequent to the application of the coating have at least one of a second property of adhesiveness less than the first property of adhesiveness and a second property of friction less than the first property of friction when in contact with each other. The system may also include a covering such as one made from a polymeric material. The covering may include a polyurethane polymer. The powder coating may be disposed on the polymeric material. The covering may include a polyetherurethane urea and a surface modifying agent. Alternatively, the system may include a graft material, such as a polyurethane polymer graft material. The powder coating may be disposed on the polymeric graft material. The graft material may include a polyetherurethane urea and a surface modifying agent. The fine powder coating may include particles of less than about 10 µm in size. In certain embodiments, the luminal surface of the stent may be coated with the fine powder coating. In one embodiment, the frame may include a stent.

According to a second aspect of the present invention, there is provided a method of manufacturing a medical device system, comprising: providing a sheath having an abluminal surface and a luminal surface; providing an expandable device, the device having an abluminal surface and a luminal surface defining a lumen; applying a coating compound comprising a powder of sodium bicarbonate, sodium maleate, sodium gluconate, sodium fumarate, sodium chloride, magnesium bicarbonate and potassium bicarbonate, on at least one of the abluminal surface of the frame and the luminal surface of the delivery instrument, and disposing the device at least partially within the sheath so that the device is at least partially in contact with the luminal surface of the sheath, wherein the device and the sheath prior to the application of the coating have at least one of a first property of adhesiveness and a first property of friction when in contact with each other, and subsequent to the application of the coating have at least one of a second property of adhesiveness less than the first property of adhesiveness and a second property of friction less than the first property of friction when in contact with each other.

In another embodiment, the invention relates to a method of manufacturing a medical device system. The method includes the steps of providing a delivery instrument comprising a sheath having an abluminal surface and a luminal surface; providing a radially-expandable frame, the frame having an abluminal surface and a luminal surface defining a substantially cylindrical lumen; applying a coating compound selected from the group consisting of sodium bicarbonate, sodium maleate, sodium gluconate, and sodium fumarate on at least one of the abluminal surface of the frame and the luminal surface of the delivery instrument, and disposing the frame at least partially within the sheath so that the frame is at least partially in contact with the luminal surface of the sheath. The coefficient of friction between the frame and the sheath subsequent to the application of the coating is less than the coefficient of friction between the frame and the sheath prior to the application of the coating, and in the range of from about 0.2 to about 0.5. The frame and the sheath prior to the application of the coating have at least one of a first property of adhesiveness and a first property of friction when in contact with each other, and subsequent to the application of the coating have at least one of a second property of adhesiveness less than the first property of adhesiveness and a second property of friction less than the first property of friction when in contact with each other. In the method, the step of applying may include dusting at least one of the abluminal surface of the frame or the luminal surface of the delivery instrument with a fine powder form of the coating compound; rolling the abluminal surface of the frame over a fine powder coating distributed on a smooth surface; evaporating an aqueous solution comprising the coating compound from the at least one of the abluminal surface of the frame or the luminal surface of the delivery instrument; and/or electrospraying solution comprising the coating compound onto the at least one of the abluminal surface of the frame or the luminal surface of the delivery instrument. The expandable device may be a stent, in which case the method may comprise the step of inserting the stent into the sheath in less than 60 minutes.

In yet another embodiment, the invention relates to a method of loading a stent into a delivery instrument. The method includes the steps of disposing a fine powder coating, selected from the group consisting of sodium bicarbonate, sodium maleate, sodium gluconate, and sodium fumarate, on at least one of an abluminal surface of the frame and a luminal surface of a delivery instrument and inserting the frame into the delivery instrument in less than 60 minutes. However, inserting may be completed most often less than 15 minutes; even less than 5 minutes; or even less 1 minute. The coefficient of friction between the frame and the sheath subsequent to the application of the coating is less than the coefficient of friction between the frame and the sheath prior to the application of the coating, and in the range of from about 0.2 to about 0.5. The frame and the sheath prior to the application of the coating have at least one of a first property of adhesiveness and a first property of friction when in contact with each other, and subsequent to the application of the coating have at least one of a second property of adhesiveness less than the first property of adhesiveness and a second property of friction less than the first property of friction when in contact with each other. The coating may be applied to the frame, the frame being in either compressed or expanded configuration.

### Brief Description of the Drawings

FIG. 1A is a side view of an exemplary medical device system of the present invention.
FIG. 1B is a side view of an exemplary medical device.
FIG. 1C is a side view of an exemplary self expanding stent-graft.
FIGS. 1D-F are cross sectional views along A-A' shown in FIG. 1A.
FIGS. 2A-B are electron micrographs of bicarbonate dusted THORALON stent.
FIGS. 3A-B are electron micrographs of ground sodium bicarbonate.
FIGS. 4A-D are photomicrographs of stents before (A and B) and after (C and D) dusting with sodium bicarbonate.
FIG. 5 is a graphical illustration of Example 4.
FIG. 6 is a graphical illustration of Example 5.

### Best Mode For Currying Out The Invention [or Mode(s) / if applicable]

The following detailed description and appended drawings describe and illustrate various exemplary embodiments of the invention. The description and drawings serve to enable one skilled in the art to make and use the invention.

### Definitions

It should be understood that the terms "a" and "an" as used above and elsewhere herein refer to "one or more" of the enumerated components. For example, "a" polymer refers to one polymer or a mixture comprising two or more polymers.

The recitation of "about" or "substantially" used with reference to a quantity, such as an angle; level; value; dimension; size; or amount and includes variations in the recited quantity, level, value, dimension, size, or amount that are equivalent to the quantity, level, value, dimension, size, or amount recited, for instance an amount that is insubstantially different from a recited quantity, level, value, dimension, size for an intended purpose or function.

The term "biocompatible" refers to a material that is substantially non-toxic in the *in vivo* environment of its intended use, and that is not substantially rejected by the patient's physiological system (i.e., is non-antigenic). This can be gauged by the ability of a material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1, entitled "Use of International Standard ISO-10993, Biological Evaluation of Medical Devices Part-1: Evaluation and Testing." Typically, these tests measure a material's toxicity, infectivity, pyrogenicity, irritation potential, reactivity, hemolytic activity, carcinogenicity and/or immunogenicity. A biocompatible structure or material, when introduced into a majority of patients, will not cause an undesirably adverse, long-lived or escalating biological reaction or response, and is distinguished from a mild, transient inflammation which typically accompanies surgery or implantation of foreign objects into a living organism.

As used herein, the term "body vessel" means any body passage lumen that conducts fluid, including but not limited to blood vessels, esophageal, intestinal, biliary, urethral and ureteral passages.

The term "coating," as used herein and unless otherwise indicated, refers generally to material, such as bicarbonate, attached to, associated with or coated per se on a medical device. A coating can include material covering or coating entire or any portion of a medical device, and can be configured as one or more coating layers. A coating can have a substantially constant or a varied thickness and composition. Coatings can be adhered to any portion or element of a medical device surface, including the luminal surface, the abluminal surface, or any portions or combinations thereof.

When coated, the coating may be present on any portion of a surface(s) of the device. In one embodiment, the surface is the luminal (inner) surface. In another embodiment, the surface is the abluminal (outer) surface. In one embodiment, the layer covers at least about 10% of the surface. In another embodiment, the layer covers at least about 20% of the surface. In another embodiment, the layer covers at least about 30% of the surface. In another embodiment, the layer covers at least about 40% of the surface. In another embodiment, the layer covers at least about 50% of the surface. In another embodiment, the layer covers at least about 60% of the surface. In another embodiment, the layer covers at least about 70% of the surface. In another embodiment, the layer covers at least about 80% of the surface. In another embodiment, the layer covers at least about 90% of the surface. In another embodiment, the layer covers about 100% of the surface.

As used herein the terms "comprise(s)," "include(s)," "having," "has," "contain(s)," and variants thereof, are intended to be open-ended transitional phrases, terms, or words that do not preclude the possibility of additional acts or structure.

As used herein, "endolumenally," "intraluminally" or "transluminal" all refer synonymously to implantation placement by procedures where the medical device is advanced within and through the lumen of a body vessel from a remote location to a target site within the body vessel. In vascular procedures, a medical device will typically be introduced "endovascularly" using a catheter over a wire guide under fluoroscopic guidance. The catheters and wire guides may be introduced through conventional access sites to the vascular system.

The terms "luminal surface" or "luminal side," as used herein, refer to the portion of the surface area of a medical device or a delivery instrument defining at least a portion of an interior lumen. Conversely, the term "abluminal surface" or "abluminal side," as used herein, refers to portions of the surface area of a medical device or a delivery instrument that do not define at least a portion of an interior lumen. For example, where the medical device is a tubular frame formed from a plurality of interconnected struts and bends defining a cylindrical lumen, the abluminal surface can include the exterior surface, sides and edges of the struts and bends, while the luminal surface can include the interior surface of the struts and bends. For example, where the medical device is a stent-graft, the abluminal surface can include the exterior surface of the graft material, while the luminal surface can include the interior surface of the graft material.

The terms "frame" and "support frame" are used interchangeably herein to refer to a structure that can be implanted or adapted for implantation, within the lumen of a body vessel and that can be used to hold tissue in place within a body, including an interior portion of a blood vessel. In certain embodiments, the frame may be a stent.

As used herein, the terms "stent" or "stent element" refer to any structure that can be used to hold tissue in place within a body, including an interior portion of a blood vessel, lymph vessel, ureter, bile duct or portion of the alimentary canal. A stent may be useful for opening up blood vessels, such as for example, an artery, vein or capillary thereby improving blood flow; keeping an artery, vein or capillary open; sealing any tears or openings in an artery, vein or capillary; preventing an artery, vein or capillary wall from collapsing or closing off again; or preventing small pieces of plaque from breaking off. In one embodiment, the stent is a stent-graft.

A "stent-graft," as used herein, refers to a device where a section of a graft material (i.e., tubular element) is supported by at least one stent element. The graft material can be any biocompatible synthetic (e.g., ePTFE, DACRON, THORALON) or natural (i.e., biologically-derived) material. The stent-graft can include a single or multiple tubular elements. For example, a stent-graft including a single tubular element can be used for treating thoracic aortic aneurysm; a stent-graft including multiple tubular elements can be configured to form a bifurcated device for treating abdominal aortic aneurysm. The stent element(s) may be balloon-expandable or self-expandable and may or may not be interconnected. The term also encompasses grafted stents, where the stent is covered partially or in its entirety with a natural or synthetic graft material (e.g., ZENITH stent from Cook, Inc.). In one embodiment, the stent-graft is a prosthetic. The stent-graft can be formed by taking a graft material and affixing stents to the graft material.

The term "graft material" as used herein refers to a biocompatible flexible material that can be attached to a support frame, for example to form a stent-graft. A graft material can have any suitable shape, but preferably forms a tubular prosthetic vessel. According to this invention, a graft material can be formed from any suitable biocompatible material, having the property of adhesiveness or tackiness when placed in high pressure contact with a smooth surface and the property of folding over during loading into a deployment device. One example of such material is THORALON.

The term "covering" refers to a layer of material, preferably a polymeric material that can be applied to a stent. The covering functions to, for example, prevent hemorrhage, occlude an aneurysm or prevent tissue in-growth. One example of a material that may be used as the covering includes THORALON.

The term "covered stent" refers to a stent that includes at least one layer of a covering including polymeric material. A covered stent can be formed by taking a stent and applying the covering to the stent.

The terms "delivery system," "delivery device," or "delivery instrument" mean a device used to deliver and place at the delivery site the medical device of this invention. The delivery system includes, among other elements, a delivery sheath.

The invention relates to the use of lubricant compounds including bicarbonates, such as sodium bicarbonate, and other compounds including sodium maleate, sodium gluconate, and sodium fumarate as a coating for intraluminal medical devices, such as stents, stent-grafts and covered stents as well as the appropriate delivery instruments used to deliver the medical devices. These compounds act as lubricants to advantageously improve the process of loading of the medical devices into suitable delivery instruments yet are a non-toxic substances that do not cause adverse reactions in animal or human subjects. These materials also may aid in lowering the deployment force of medical devices so that a physician does not need to apply a significant force to deploy the device within a body lumen.

### Coatings

Lubricant compounds including bicarbonates, such as sodium bicarbonate, and other compounds including sodium maleate, sodium gluconate, and sodium fumarate, magnesium bicarbonate, or potassium bicarbonate may be used as a coating for intraluminal medical devices, such as stents, stent-grafts and covered stents, and/or delivery instruments used to deliver the medical devices, according to this invention as long as these coatings are non-toxic and blood compatible. Preferably, the coating is a bicarbonate coating, such as sodium bicarbonate coating. One significant advantage of using sodium bicarbonate as a coating is that sodium bicarbonate is a natural component of the blood and disassociates into sodium ions and bicarbonate ions. Similar advantages are associated with other sodium salts. Sodium chloride can also be used, but care needs to be taken regarding long-term storage if it is used in combination with certain graft materials.

Similar advantages are associated with other bicarbonate salts.

A mixture of one or more of the above compounds may be used for the powder coating.

### Bicarbonate coating

The lubricious bicarbonate coating includes sodium bicarbonate that is readily dissolved within the body vessel as the stent-graft 10 is being deployed from a catheter delivery system. Sodium bicarbonate or sodium hydrogen carbonate is the chemical compound with the formula NaHCO₃. Sodium bicarbonate is a white solid that is crystalline but often appears as a fine powder. Advantages of using sodium bicarbonate as a coating material for medical devices include its durability, solid formulation, flexibility at room temperature, water solubility and ability to dissolve readily when exposed to blood under normal blood temperatures and pH without any detrimental systemic side effects or toxicity to a patient.

Preferably, sodium bicarbonate is in a form of a finely ground or otherwise produced powder (particles of size less than about 10 µm) that will form a fine powder coating. However, other particle sizes larger than 10 µm may also be suitable for coating of the medical devices. Methods of producing sodium bicarbonate powder, sizes and shapes of the bicarbonate particles were previously provided in U.S. Pat. No. 5,645,840. For example, sodium bicarbonate powder can be obtained in the form of cohesive agglomerated crystallites of primary particles. The agglomerated crystallites can have an average diameter between about 1-10 microns. One exemplary method of preparation involves the dissolution of alkali metal bicarbonate in water at 20°-60°C, and the subsequent addition of a water- miscible organic solvent to the aqueous solution to precipitate primary particles of sodium bicarbonate, which aggregate to form cohesive agglomerated crystallites. The average size of the primary particles typically is about 0.5-2 microns, and the average agglomerated crystallite size is 4-12 microns. Other methods may also be employed and are known in the art.

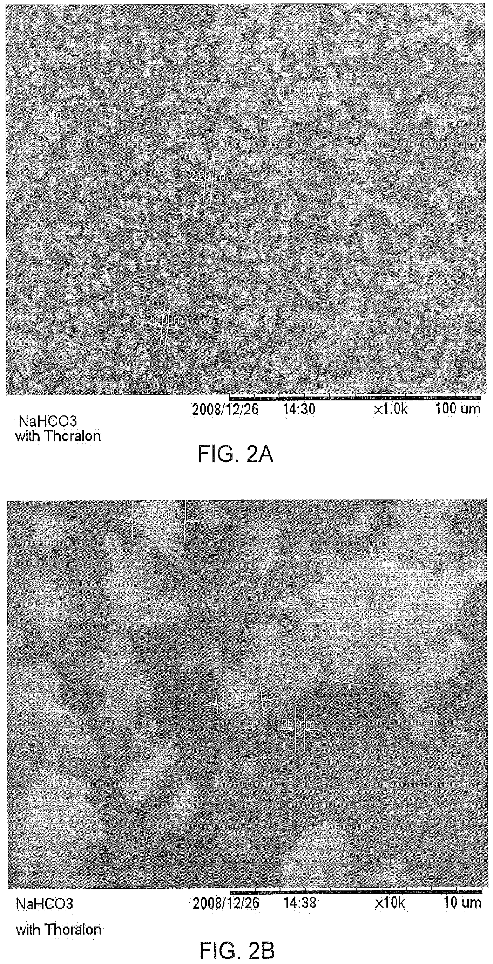

The particles may have a specified shape, such as spherical, square, etc. or be of an unspecified shape. A combination of both types of particle shapes may also be used. See FIGS. 2A-4D.

The amount of the lubricious sodium bicarbonate in the coating may be selected based on the size of the device as well as the size of the delivery instrument used for the delivery of the device. The total amount of a lubricious bicarbonate such as sodium bicarbonate applied to the outer surface of the device and/or luminal surface of the delivery instrument is preferably provided in an amount that permits easy and quick crimping of the coated device to a desired radially compressed diameter and easy and quick loading into a delivery instrument as well as easy deployment of the device. In addition, the amount of the lubricious bicarbonate is preferably selected to permit the device to be expanded from the radially compressed configuration within a body vessel and, in certain embodiments, to subsequently release any therapeutic agents included with the device at a desired rate. Preferably, the amount of lubricious bicarbonate is selected so as to provide adequate protection against physical damage to the graft material or the polymeric coating during crimping, loading, and expansion of the stent-graft or covered stent, respectively, without undesirably altering any other properties of the device, such as the rate of release of any therapeutic agents that may be included with the device within a body vessel at an intended point of treatment.

Preferably, the lubricious coating would be about 1 bicarbonate particle thick. Any surface of the device may be completely or partially coated with the lubricious bicarbonate coating, resulting in the thickness of the bicarbonate coating of about 1 particle or less, in the instance of partial coating of the device's surface.

### Device Configurations

Generally, the present disclosure describes medical devices and systems for placement within a body passage.

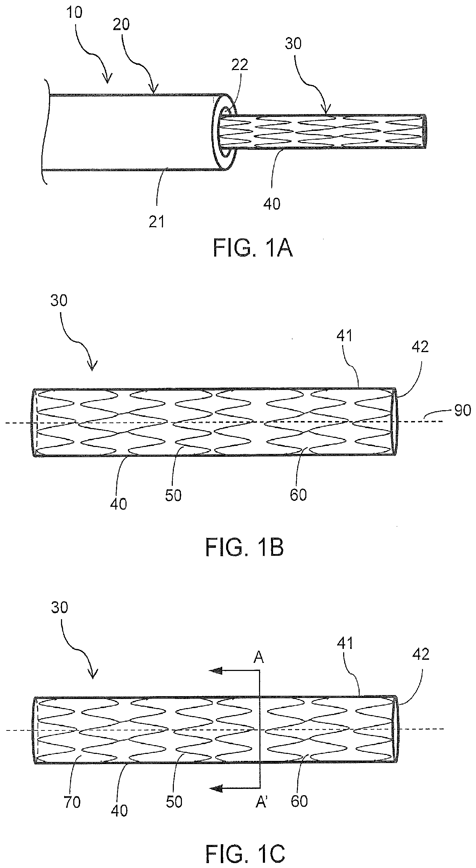

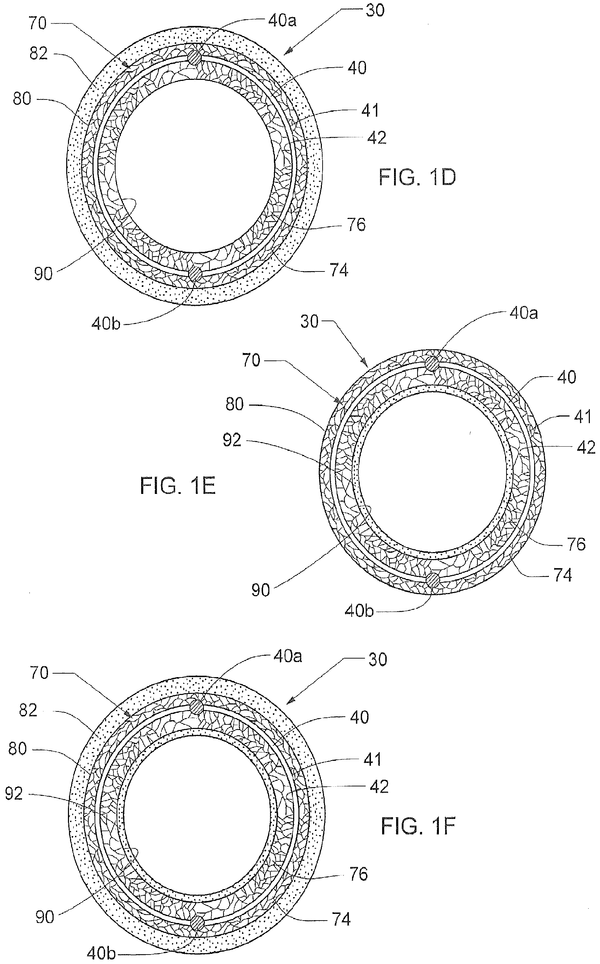

Referring to FIG. 1A, a medical device system **10** of the present invention includes a delivery instrument, such as a sheath **20** having an abluminal surface **21** and a luminal surface **22;** and a radially expandable medical device **30,** such as a stent, stent-graft or a covered stent (shown in the collapsed configuration) that is disposed at least partially within the sheath **20.**

The medical device **30** is further coated with a lubricious coating, such as sodium bicarbonate coating on at least a portion of at least one surface of the device **30.** In addition or alternatively, at least a portion of at least one surface of the delivery instrument **20** may be coated with the lubricious coating.

The medical device **30** can also optionally include a releasable therapeutic agent.

Typically, the device **30** has a cylindrical shape formed by at least a plurality of longitudinally-aligned sinusoidal ring members (e.g., stents) forming a support frame **40.** The frame **40** is radially-expandable and may be a self-expandable or balloon-expandable. The frame **40** can be formed from any suitable structure that can maintain an attached graft material or covering in a desired position, orientation or range of motion to perform a desired function. Preferably, the frame **40** is a radially self-expandable frame adapted for implantation within a body vessel from a delivery instrument.

In one embodiment, referring to FIG 1B, the device **30** includes a frame **40.** The frame **40** may be formed, for example, by eight self-expanding sinusoidal ring members **50** axially aligned around a longitudinal axis to form a cylindrical shape. The sinusoidal ring members **50** are optionally joined by longitudinal struts. The frame **40** includes an abluminal surface **41** and the luminal surface **42** that define a substantially cylindrical lumen of the device.

In certain embodiments, at least a portion of the abluminal surface **41** of the support frame **40** is coated with a lubricious bicarbonate compound, such as sodium bicarbonate to form a lubricious coating **60** that reduces the frictional force, resulting in a lower coefficient of friction of the device **30** during loading of the device **30** into a delivery instrument as compared to a much higher coefficient of friction of device that is uncoated with the bicarbonate compound.

In another embodiment, referring to FIG. 1C, the device **30** may include a tubular graft material **70** affixed to the frame **40.** Methods of affixing or attaching graft materials to support frames are well known in the art and include suturing, gluing, embedding, etc.

In certain embodiments, the graft material **70** encloses the support frame **40.** FIG. 1D is a lateral cross section along the line A-A' of the medical device **30** shown in FIG. 1C. The graft material **70** preferably includes an inner portion **76** positioned on the luminal side **42** of the support frame **40,** and an outer portion **74** positioned on the abluminal side **41** of the support frame **40.** The inner portion **76** refers to the portion of the graft material **70** positioned on the luminal side of the center of the support frame **40**; the outer portion **74** refers to the graft material **70** positioned on the abluminal side of the center of the support frame **40.** Support frame portions **40a**, **40b** may be positioned in the middle of a single layer of the graft material **70**, or between two layers of the graft material **70.** Preferably, the graft material **70** is formed by positioning the support frame **40** around the inner portion **76** of the graft material **70** and then contacting the outer portion **74** of the graft material with the abluminal surface **41** of the support frame **40** under conditions that join the inner portion **76** and the outer portion **74** of the graft material **70** to each other through openings in the support frame **40**. The inner portion **76** and the outer portion **74** of the graft material **70** may have the same or different compositions or structures, and may form portions of a single layer or form separate layers. Optionally, the inner portion **76** and/or outer portion **74** of the graft material include multiple layers of material having different compositions and/or different structures.

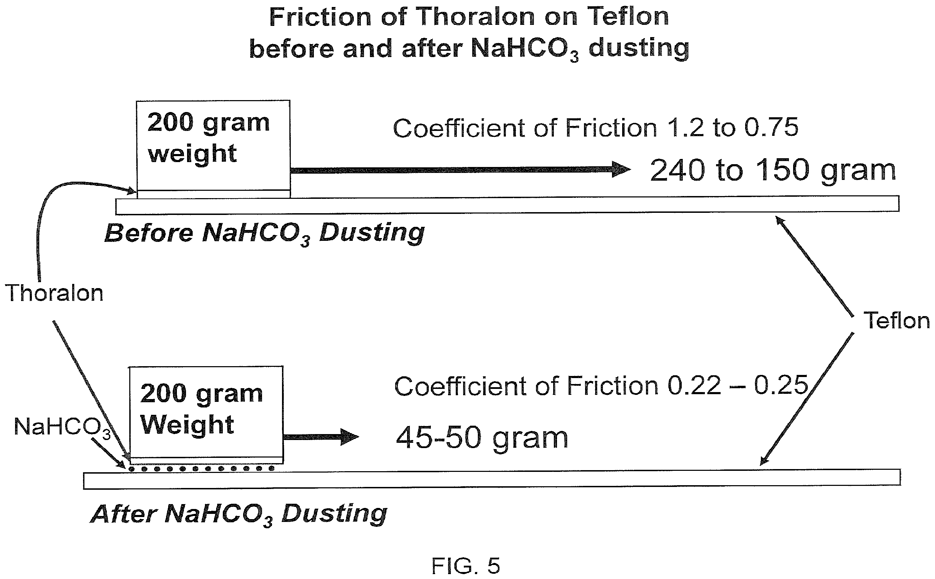

In one embodiment, further referring to FIG. 1D, at least a portion of the abluminal surface **80** of the stent-graft **30** is coated with a lubricious bicarbonate compound, such as sodium bicarbonate to form a lubricious coating **82** that reduces the frictional force and adhesiveness, resulting in a lower coefficient of friction of the graft material **70** during crimping and loading of the device **30** into a delivery instrument as compared to a much higher coefficient of friction of a graft material that is uncoated with the bicarbonate compound. For example, coefficient of friction was shown to be significantly reduced from 1.2 to 0.75 for an uncoated stent-graft to 0.22 to 0.25 for a bicarbonate coated stent-graft (Example 4; FIG. 5).

In another embodiment shown in FIG. 1E, at least a portion of the luminal surface **90** of the graft material **70** is coated with a lubricious bicarbonate compound, such as sodium bicarbonate to form a lubricious coating 92 that reduces the frictional force of the device.

In yet another embodiment shown in FIG. 1E, at least a portion of the abluminal **80** and the luminal **90** surfaces of the graft material **70** are coated with a lubricious bicarbonate compound, such as sodium bicarbonate to form lubricious coatings **82**, and **92** that reduces the frictional force of the device.

Alternatively, or in addition to the coating of the device, the luminal surface of the delivery instrument that includes a delivery sheath may be coated with a lubricious bicarbonate compound (not shown), such as sodium bicarbonate to form a lubricious coating that reduces the frictional force between the device and the delivery instrument.

### Support Frame

The support frame **40** preferably defines a substantially cylindrical or elliptical lumen providing a conduit for fluid flow. The frame structure may comprise a plurality of struts, which can be of any suitable structure or orientation. In some embodiments, the frame comprises a plurality of struts connected by alternating bends. For example, the frame can be a ring or annular tube member comprising a series of struts in a "zig-zag" pattern. The frame can also comprise multiple ring members with struts in a "zig-zag" pattern, for example by connecting the ring members end to end, or in an overlapping fashion. In some embodiments, the struts are substantially aligned along the surface of a tubular plane, and substantially parallel to the longitudinal axis of the support frame. Support frames can also be formed from braided strands of one or more materials, helically wound strands, ring members, consecutively attached ring members, tube members, and frames cut from solid tubes. The support frame is preferably selected for an intended site of implantation, such as placement to treat an aneurysm. For example, a ZILVER intravascular stent (Cook Inc., Bloomington, Ind.) may be used. In one example, the frame has a diameter in a radially expanded configuration of about 9-10 mm and a length of about 40 mm-80 mm. A suitable graft material, such as a biocompatible polyurethane, is preferably adhered to the luminal and abluminal surfaces of the frame.

The specific implantable frame chosen will depend on several considerations, including the size and configuration of the vessel and the size and nature of the medical device. The frame can perform any desired function, including a stenting function. The frame configuration may be selected based on several factors, including the vessel in which the medical device is being implanted, the axial length of the treatment site, the inner diameter of the body vessel, and the desired delivery method for placing the support structure. Those skilled in the art can determine an appropriate stent based on these and other factors. The implantable frame can be sized so that the expanded configuration is slightly larger in diameter that the inner diameter of the vessel in which the medical device will be implanted. This sizing can facilitate anchoring of the medical device within the body vessel and maintenance of the medical device at a point of treatment following implantation. Preferably, the support frame has an expanded inner diameter of about 5 mm to about 46 mm, more preferably about 4 mm to about 8 mm and most preferably about 6 mm. The support frame can have any suitable length. The length of the support frame is selected based on the desired site of implantation. Examples of suitable frame lengths include frames with a length of about 10 to 300 mm long, more preferably about 20-100 mm and most preferably about 40-80 mm. However, this application is not limited to the specified sizes of the support frames. Any and all sizes of available support frames are included.

The implantable frame may be formed from any suitable biocompatible material that allows for desired therapeutic effects upon implantation in a body vessel. Examples of suitable materials include, without limitation, any suitable metal or metal alloy, such as: stainless steels (e.g., 316, 316L or 304), nickel-titanium alloys including shape memory or superelastic types (e.g., nitinol or elastinite); inconel; noble metals including copper, silver, gold, platinum, palladium and iridium; refractory metals including molybdenum, tungsten, tantalum, titanium, rhenium, or niobium; stainless steels alloyed with noble and/or refractory metals; magnesium; amorphous metals; plastically deformable metals (e.g., tantalum); nickel-based alloys (e.g., including platinum, gold and/or tantalum alloys); iron-based alloys (e.g., including platinum, gold and/or tantalum alloys); cobalt-based alloys (e.g., including platinum, gold and/or tantalum alloys); cobalt-chrome alloys (e.g., elgiloy); cobalt-chromium-nickel alloys (e.g., phynox); alloys of cobalt, nickel, chromium and molybdenum (e.g., MP35N or MP20N); cobalt-chromium-vanadium alloys; cobalt-chromium-tungsten alloys; platinum-iridium alloys; platinum-tungsten alloys; magnesium alloys; titanium alloys (e.g., TiC, TiN); tantalum alloys (e.g., TaC, TaN); L605; bioabsorbable materials, including magnesium; or other biocompatible metals and/or alloys thereof.

In some embodiments, the implantable frames impart radially outward directed force during deployment, whether self-expanding or radially-expandable. The radially outward directed force can serve to hold the body lumen open against a force directed radially inward, as well as preventing restriction of the passageway through the lumen by intimal flaps or dissections generated by actions, such as prior balloon angioplasty. Another function of the radially outward directed force can also fix the position of the stent within the body lumen by intimate contact between the stent and the walls of the lumen. Preferably, the outwardly directed forces do not traumatize the lumen walls. Preferably, the frame material is capable of significant recoverable strain to assume a low profile for delivery to a desired location within a body lumen. After release of the compressed self-expanding resilient material, it is preferred that the frame be capable of radially expanding back to its original diameter or close to its original diameter. Accordingly, some embodiments provide frames made from material with a low yield stress (to make the frame deformable at manageable balloon pressures), high elastic modulus (for minimal recoil), and is work hardened through expansion for high strength. Particularly preferred materials for self-expanding implantable frames are shape memory alloys that exhibit superelastic behavior, i.e., are capable of significant distortion without plastic deformation. Frames manufactured of such materials may be significantly compressed without permanent plastic deformation, i.e., they are compressed such that the maximum strain level in the resilient material is below the recoverable strain limit of the material. Other embodiments provide frames that are not self-expanding, or that do not comprise superelastic materials. Preferably, the implantable frame comprises a self-expanding nickel titanium (NiTi) alloy material, stainless steel or a cobalt-chromium alloy.

Preferably, the support frame **40** is self-expanding. Upon compression, self-expanding frames can expand toward their pre-compression geometry. In some embodiments, a self-expanding frame can be compressed into a low-profile delivery conformation and then constrained within a delivery system for delivery to a point of treatment in the lumen of a body vessel. At the point of treatment, the self-expanding frame can be released and allowed to subsequently expand to another configuration. Discussions relating to nickel titanium alloys and other alloys that exhibit behaviors suitable for frames can be found in, e.g., U.S. Pat. No. 5,597,378 and WO 95/31945. A preferred shape memory alloy is Ni--Ti, although any of the other known shape memory alloys may be used as well. Such other alloys include: Au--Cd, Cu--Zn, In--Ti, Cu--Zn--Al, Ti--Nb, Au--Cu--Zn, Cu--Zn--Sn, CuZn--Si, Cu--Al--Ni, Ag--Cd, Cu--Sn, Cu--Zn--Ga, Ni--Al, Fe--Pt, U-Nb, Ti--Pd--Ni, Fe--Mn--Si, and the like. These alloys may also be doped with small amounts of other elements for various property modifications as may be desired and as is known in the art, Nickel titanium alloys suitable for use in manufacturing implantable frames can be obtained from, e.g., Memry Corp., Brookfield, Conn. One suitable material possessing desirable characteristics for self-expansion is Nitinol, a Nickel-Titanium alloy that can recover elastic deformations of up to 10 percent. This unusually large elastic range is commonly known as superelasticity.

Suitable implantable frames can also have a variety of configurations, including braided strands, helically wound strands, ring members, consecutively attached ring members, tube members, and frames cut from solid tubes. Also, suitable frames can have a variety of sizes. The exact configuration and size chosen will depend on several factors, including the desired delivery technique, the nature of the vessel in which the device will be implanted, and the size of the vessel. A frame structure and configuration can be chosen to facilitate maintenance of the device in the vessel following implantation. The implantable frame can be formed in any suitable shape, including a ring, a stent, a tube, or a zig-zag configuration. In one embodiment, the implantable frame can be self-expanding or balloon-expandable.

### Graft Material

Graft material **70** can include a graft polymer. Preferably, the graft material is a biocompatible polyurethane material. Preferably, the graft material is a biocompatible polyurethane material comprising a surface modifying agent, as described herein. These types of material have the property of adhesiveness or tackiness when placed in high pressure contact with a smooth surface and the property of folding over during loading into a deployment instrument. The terms "adhesiveness" or "tackiness" when referring to the property of the graft material mean that the graft material is adhesive and sticky.

The graft material may be selected from a variety of materials, but preferably comprises a biocompatible polyurethane material. One particularly preferred biocompatible polyurethane is THORALON (THORATEC, Pleasanton, Calif.), described in U.S. Pat. Application Publication No. 2002/0065552 A1 and U.S. Pat. No. 4,675,361. The biocompatible polyurethane material sold under the tradename THORALON is a polyurethane base polymer (referred to as BPS-215) blended with a siloxane containing surface modifying additive (referred to as SMA-300). The concentration of the surface modifying additive may be in the range of 0.5% to 5% by weight of the base polymer. Other suitable graft materials, such as Dacron may also be incorporated.

THORALON and other polyurethane polymer materials, in addition to silicone rubber polymers, exhibit high amount of adhesiveness or friction toward other materials. In other words, these materials may be characterized as having the property of adhesiveness when placed in high pressure contact with a smooth surface and, as a result also the property of folding over of the graft during loading into a delivery device. This is especially evidenced when these polymers are placed in high pressure contact with another smooth surface such as nylon, PTFE, metal, glass, etc., and then an attempt is made to slide on of these materials with respect to the other. The attempt is unsuccessful. Specifically, a vascular stent that is covered with THORALON on its outer surface can be difficult to radially compress and load into a sheath because of this friction. For example, on average, the loading time for a 7 inch-long aortic arch stent-graft that is not coated with bicarbonate coating may be several hours.

Biocompatible polyurethane polymers have been used in certain vascular applications and are characterized by thromboresistance, high tensile strength, low water absorption, low critical surface tension, and good flex life. For example, the biocompatible polyurethane material sold under the tradename THORALON is believed to be biostable and to be useful *in vivo* in long term blood contacting applications requiring biostability and leak resistance. Because of its flexibility, THORALON is useful in larger vessels, such as the abdominal aorta, where elasticity and compliance is beneficial. The SMA-300 component (THORATEC) is a polyurethane comprising polydimethylsiloxane as a soft segment and the reaction product of diphenylmethane diisocyanate (MDI) and 1,4-butanediol as a hard segment. A process for synthesizing SMA-300 is described, for example, in U.S. Pat. Nos. 4,861,830 and 4,675,361. The BPS-215 component (THORATEC) is a segmented polyetherurethane urea containing a soft segment and a hard segment. The soft segment is made of polytetramethylene oxide (PTMO), and the hard segment is made from the reaction of 4,4'- diphenylmethane diisocyanate (MDI) and ethylene diamine (ED).

Biocompatible polyurethane polymers can be formed as non-porous material or as a porous material with varying degrees and sizes of pores, as described below. Implantable medical devices can comprise one or both forms of biocompatible polyurethane polymers. The graft material preferably comprises the non-porous form of the biocompatible polyurethane sold as THORALON. The porous forms of biocompatible polyurethane polymers can also be used as a graft material, but are preferably employed as an adhesion promoting region.

Graft materials other than THORALON but also having the property of adhesiveness when placed in high pressure contact with a smooth surface and the property of folding over during loading into a deployment device, would also benefit from having a lubricious sodium bicarbonate coating to improve loading of the medical device into a suitable delivery device, and are also included in this application.

Referring to FIGS. 1D-F, the graft material **70** may be preferably formed by one or more layers of a biocompatible polyurethane. The inner portion **76** and/or the outer portion **74** may include one or more layers, of biocompatible polyurethane each having a different composition and/or structure. For example, the graft material **70** may include a non-porous or porous polyetherurethane composition with a siloxane surface modifying additive.

The thickness of the graft material **70** may be selected to provide the desired mechanical properties, such as a suitable durability to the graft material **70** or a desired minimum radius upon radial compression of the stent-graft **30** after crimping, or optionally, desired loading of any therapeutic agents. The inner portion **76** preferably includes two layers: an inner layer comprising a porous biocompatible polyurethane, and an outer layer including a non-porous biocompatible polyurethane. The inner layer defines the lumen of the medical device **30** and the outer layer contacts the support frame **40.** The thickness of the outer layer of the inner portion **76** is typically 1.5-3.0 times thicker than the inner layer of the inner portion **76.** The outer portion **74** of the graft material **70** is preferably a multi-layer structure including an inner layer in contact with the support frame **40** and portions of the outer layer of the inner portion **76** through interstitial spaces in the support frame. The inner layer of the outer portion **74** preferably includes a non-porous polyurethane. The outer portion **74** further includes a second layer positioned on the abluminal surface of the inner layer and including a porous polyurethane composition. The inner layer of the outer portion **74** is typically 1.5-3.0 times thicker than the second layer of the outer portion **74.** The second layer may form the abluminal surface of the graft material **70,** or the outer portion **74** may further include a third layer formed from a non-porous polyurethane material. The third layer of the outer portion **74** preferably forms the abluminal surface of the graft material **70,** and is preferably about 1 to 3 times the thickness of the second layer of the outer portion **74.**

### Methods of Manufacture, Loading and Delivery

Methods of making the support frame were provided above and are known in the art.

The graft is preferably formed from a biocompatible polyurethane material, such as THORALON. The graft can be attached to an implantable support frame by drying a solution of the dissolved THORALON material onto the luminal and abluminal surfaces of the support frame. The dried polyurethane material can adhere to the support frame, or two layers of the dried polyurethane material on either side of the support frame can be attached to each other through interstitial holes in the support frame.

The graft material can be formed by at least one of three methods: (1) spraying, (2) dipping or (3) casting of the THORALON solution, and drying the polymer around portions of a support frame. Alternatively, a dried sheet of THORALON material can be adhered to a support frame using an adhesive, sutures, UV-activated polymers, melting, or any suitable means of attachment providing a desirably durable attachment between the graft material and the implantable frame. Preferably, a solution of the dissolved graft material can be coated onto a portion of the frame and attached to the frame as the solution is dried.

Once the device is ready, the device can be coated with a suitable lubricious coating, such as sodium bicarbonate coating. The bicarbonate compound may be installed on the device by various suitable methods. For example, in certain embodiments, the bicarbonate may be installed as a finely ground powder by dusting the medical device to be coated or by rolling the device to be coated over the particles of bicarbonate distributed on a smooth surface. The device may also be coated by wiping sodium bicarbonate grains on the surface of the device with a cotton or lint-free swab. Alternatively, the device may be placed in a covered beaker or a closed plastic bag that includes bicarbonate grains and shaken to dust the bicarbonate over the device. The excess grains can be removed by tapping the device gently on a hard surface prior to loading into a delivery device. Alternatively, bicarbonate coating may be deposited by evaporating an aqueous solution containing the lubricant on the surface of the item to be coated. An electrostatic potential difference also may be used in addition to any of the above-mentioned methods to produce a more thorough covering of the stent-graft. As such, in yet other instances, the bicarbonate can be dissolved in a solvent(s) and sprayed onto the medical device using any conventional spray gun, such as a spray gun manufactured by Badger (Model No. 200), an electrostatic spray gun, or most preferably an ultrasonic nozzle spray gun to produce more uniform particle distribution over the outside covering.

Other suitable methods of coating the medical device with bicarbonate compound are also contemplated.

The bicarbonate compound is preferably ground into very fine particles of less than about 10 µm. This may be achieved by grinding the bicarbonate compound in a commercially available grinder, such as a coffee grinder or jet grinder, and then further grinding the material with a mortar and pestle. See also, U.S. Pat. No. 5,645,840.

In one particular embodiment, a method of manufacturing a bicarbonate-coated medical device includes the steps of: providing a delivery instrument comprising a sheath having an abluminal surface and a luminal surface; providing a radially-expandable frame, the frame having an abluminal surface and a luminal surface defining a substantially cylindrical lumen; applying a coating compound selected from the group consisting of sodium bicarbonate, sodium maleate, sodium gluconate, and sodium fumarate on at least one of the abluminal surface of the frame and the luminal surface of the delivery instrument, and disposing the frame at least partially within the sheath so that the frame is at least partially in contact with the luminal surface of the sheath. The coating compound may be applied to the frame, which may be in a compressed configuration or may be applied to the frame prior to radially compressing the frame (expanded configuration). The coefficient of friction between the frame and the sheath subsequent to the application of the coating is less than the coefficient of friction between the frame and the sheath prior to the application of the coating, and in the range of from about 0.2 to about 0.5. The frame and the sheath prior to the application of the coating have at least one of a first property of adhesiveness and a first property of friction when in contact with each other, and subsequent to the application of the coating have at least one of a second property of adhesiveness less than the first property of adhesiveness and a second property of friction less than the first property of friction when in contact with each other.

Once the surface(s) of the medical device and/or the luminal surface of the delivery instrument is coated with the lubricious coating, the device can be easily loaded into a suitable delivery instrument, such as a catheter, by sliding the device inside the lumen of the delivery instrument. In one embodiment, the device can loaded into a delivery instrument by radially compressing and loading the frame into a delivery instrument. The sodium bicarbonate coating aids in the crimping and loading process. A restraining means may maintain the device in the radially compressed configuration. For example, a self-expanding stent-graft may be retained within a slidable sheath, while stent-grafts that are not self-expanding may be crimped over a balloon portion of a delivery catheter. The compressed stent-graft is thereby mounted on the distal tip of the delivery device, translated through a body vessel on the delivery device, and deployed from the distal end of the delivery device. The presence of the sodium bicarbonate coating will further reduce the deployment force of the device and aid in the deployment in the body vessel. Also, the presence of the lubricious bicarbonate coating allows for a fast loading in the matter of minutes, as compared to often hours of loading time for an uncoated stent-graft.

For example, a delivery device may be a catheter comprising a pushing member adapted to urge the stent-graft away from the delivery catheter. A sheath may be longitudinally translated relative to the stent-graft to permit the stent-graft to radially self-expand at the point of treatment within a body vessel. Alternatively, a balloon may be inflated to radially expand the stent-graft.

Medical devices as described herein may be delivered to any suitable body vessel, including a vein, artery, biliary duct, ureteral vessel, body passage or portion of the alimentary canal. While many preferred embodiments discussed herein discuss implantation of a medical device in a vein, other embodiments provide for implantation within other body vessels. In another matter of terminology there are many types of body canals, blood vessels, ducts, tubes and other body passages, and the term "vessel" is meant to include all such passages.

### EXAMPLES

### Example 1: Preparation of sodium bicarbonate particles

Sodium bicarbonate (NaHCO₃) crystals, which are commercially available from Spectrum Chemicals and Laboratory Products, CA and NJ were ground in a coffee grinder for 3 minutes and then further ground with a mortar and a pestle for about 5 minutes, resulting in particulate size of less than about 10 µm. The material was evaluated as follows:

Electron micrographs of bicarbonate dusted stent were taken, where the size of particles, following the grinding process is shown to be less than 10 µm (FIG. 2A-B).

### Example 2: Coating of a stent with sodium bicarbonate particles

NaHCO₃ crystals prepared as described in Example 1 were used to coat a self-expanding THORALON-coated stent weighting 227.437 grams. Specifically, the stent was coated with the sodium bicarbonate particles by dusting. The excess of sodium bicarbonate was removed by tapping the stent several times on a hard surface. The stent-graft was weighed following treatment with bicarbonate coating and the total weight of bicarbonate was 8.023 mg.

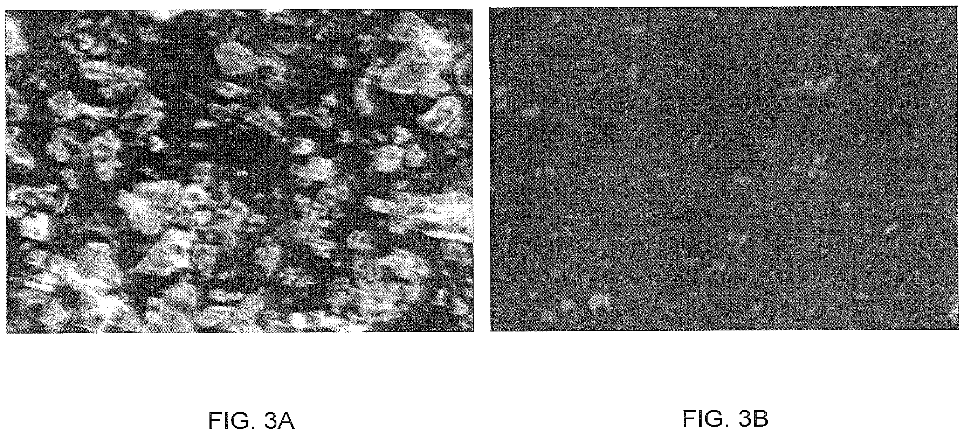

FIG. 3 shows photomicrographs taken of the bicarbonate material dusted onto a THORALON-coated stent, using unground sodium bicarbonate (A) or ground sodium bicarbonate (B). (A) and (B) are taken at the same magnification and illustrate the change in particle size resulting from the grinding method described in Example 1.

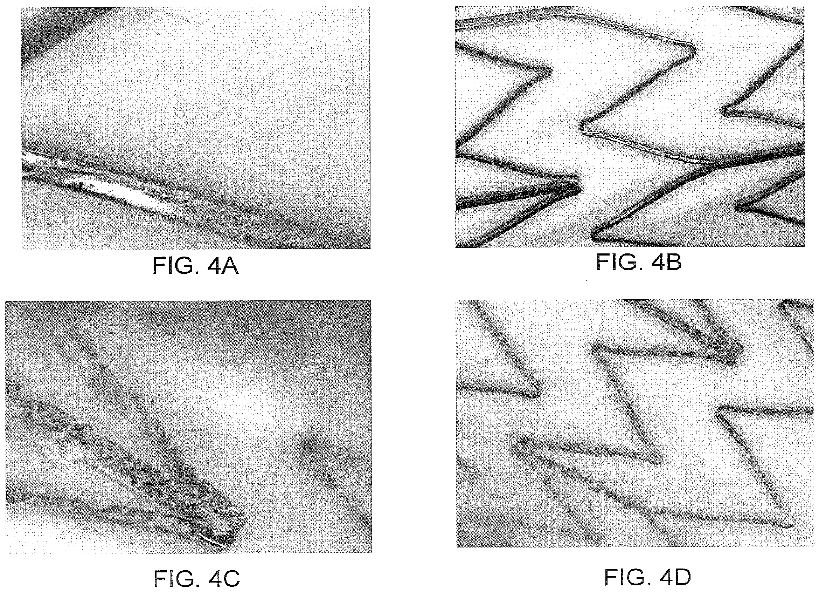

FIG. 4A-D are photomicrographs take of stents before (A and B) and after (C and D) dusting with NaHCO₃.

### Example 3: Loading of a sodium bicarbonate-covered stent-graft into a delivery device.

NaHCO₃ crystals prepared as described in Example 1 were used to coat a self-expanding 8 x 80 mm THORALON-covered stent. Specifically, the stent was coated with the sodium bicarbonate particles by dusting.

Next, the sodium bicarbonate-coated stent-graft was loaded into 7.9 F roll-sock sheath. The stent-graft slid easily into and out of the sheath. Also, the THORALON graft material did not bunch up longitudinally and fold-over and cohesively attached to the adjacent THORALON, as had occurred during previous loading attempts without the sodium bicarbonate coating. The total time for loading of the THORALON-covered stent was minutes, as compared to hours for an uncoated stent-graft. Upon removal of the stent-graft from the sheath, the stent-graft was examined and it was concluded that the process did not alter any of the stent-graft's mechanical, structural, or functional properties.

### Example 4: Properties of a bicarbonate-coated stent-graft.

NaHCO₃ crystals prepared as described in Example 1 were used to coat a self-expanding 8 x 80 mm THORALON stent-graft. Specifically, the stent-graft was coated with the sodium bicarbonate particles by dusting. Excess sodium bicarbonate was removed by tapping the stent-graft several times on a hard surface. The stent-graft was weighted before and after the dusting with sodium bicarbonate, where the total weight of bicarbonate was 8.023 mg. This is 0.2% of a typical adult incremental dose of 3696 mg per 44 meq ampule.

The coefficient of friction between THORALON and a Teflon sheet were then determined with a force transducer weight combination.

Referring to FIG. 5, the coefficient of friction was shown to be significantly reduced from 1.2 to 0.75 for an uncoated stent-graft to 0.22 to 0.25 for a bicarbonate coated stent-graft. Also, the pushing force was reduced from 240-150 gram for the uncoated stent-graft weighting 200 grams to 45-50 grams for the bicarbonate coated stent-graft of the same weight.

### Example 5:

NaHCO₃ crystals prepared as described in Example 1 were used to coat a THORALON covered weight (0.375 kg). Specifically, a THORALON covered weight was coated with the sodium bicarbonate particles by dusting (approximately one particle thick). Excess sodium bicarbonate was removed by tapping the weight several times on a hard surface.

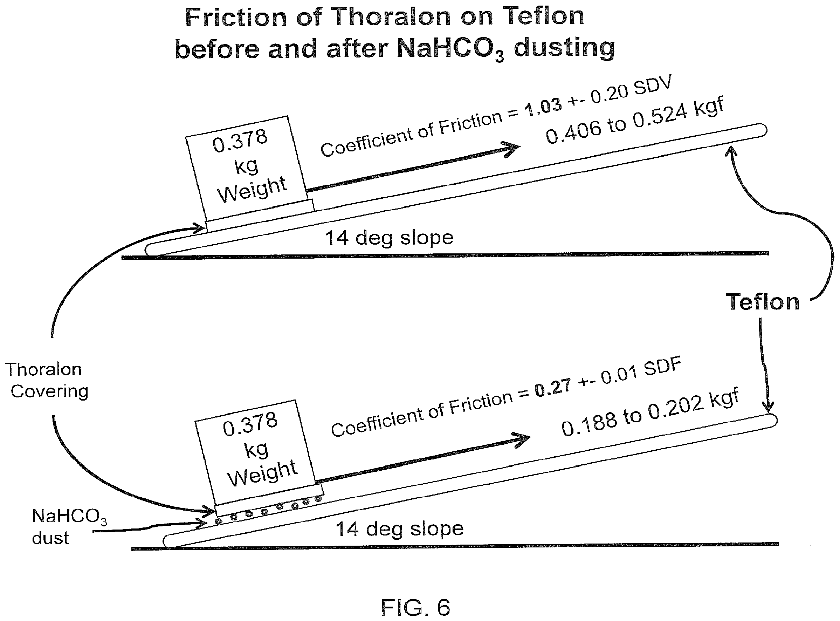

The sodium bicarbonate coated THORALON covered weight and the uncoated THORALON covered weight were then slid on the Teflon surface up the 14 degree slope as illustrated in FIG. 6. The Teflon surface was cleaned with Ethanol before the runs as indicated in Table 1 below. Each test was performed 7 times. The coefficients of friction of the coated and uncoated THORALON covered weights were calculated. The test results are provided in Table 1 below.

As noted in Table 1, the coefficient of friction was shown to be significantly reduced from 1.03 for the uncoated THORALON covered weight to 0.27 for the bicarbonate coated THORALON covered weight (p value for coefficient of friction was 4.57242 E-05).

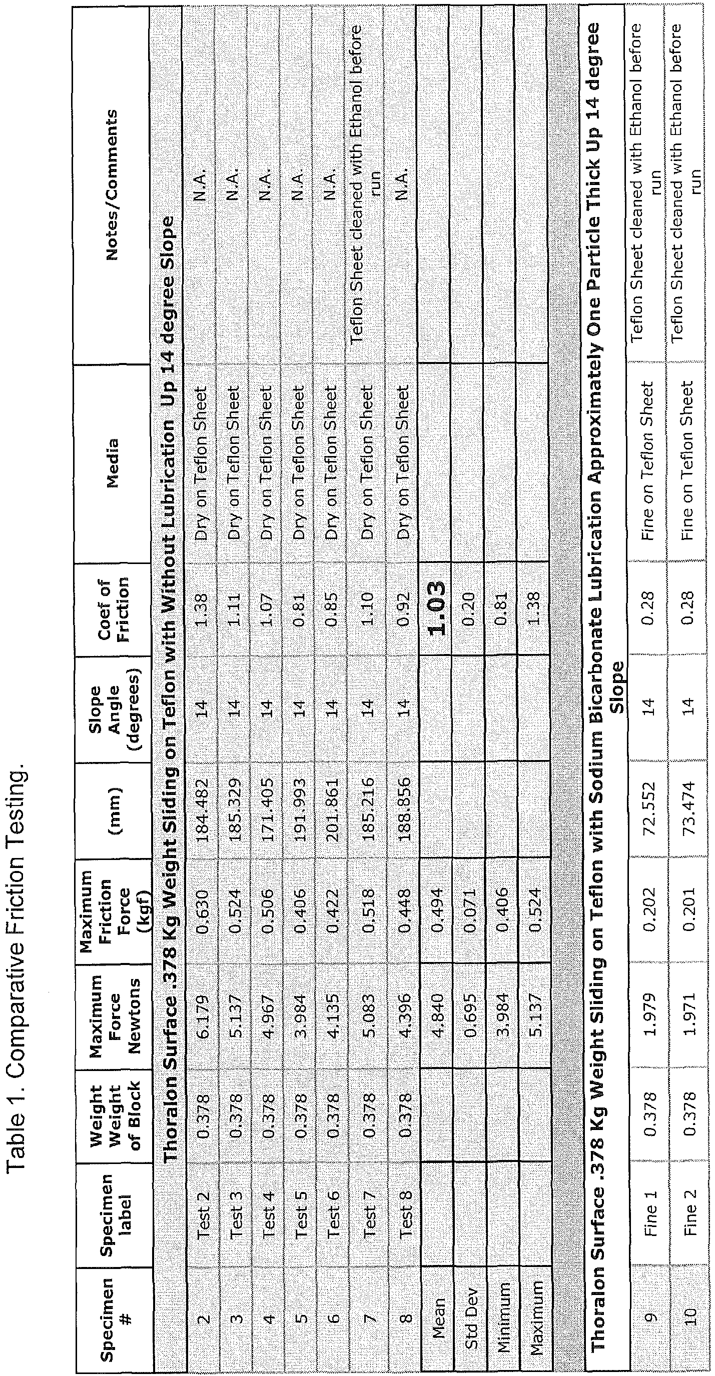

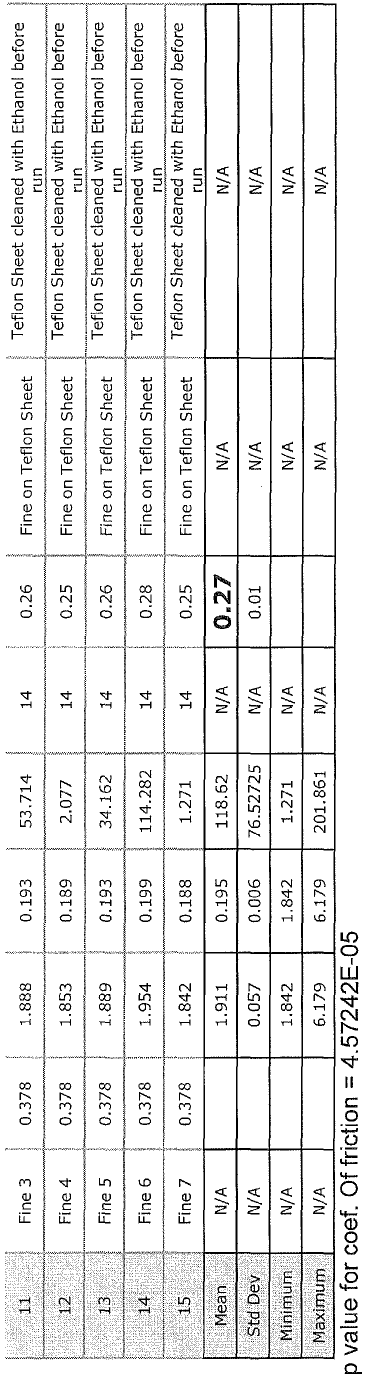

In addition to the embodiments described above, the invention includes combinations of the preferred embodiments discussed above, and variations of all embodiments.

## Claims

1. A medical device system comprising:
a sheath having an abluminal surface and a luminal surface;
an expandable medical device disposed at least partially within the sheath, the device having an abluminal surface at least partially in contact with the luminal surface of the sheath, and a luminal surface defining a lumen; and
a powder coating of one or more of sodium bicarbonate, sodium maleate, sodium gluconate, sodium fumarate, sodium chloride, magnesium bicarbonate and potassium bicarbonate, disposed on at least one of the abluminal surface of the device and the luminal surface of the sheath,
wherein the device and the sheath prior to the application of the coating have at least one of a first property of adhesiveness and a first property of friction when in contact with each other, and subsequent to the application of the coating have at least one of a second property of adhesiveness less than the first property of adhesiveness and a second property of friction less than the first property of friction when in contact with each other.

2. The system of claim 1, wherein the coefficient of friction between the device and the sheath subsequent to the application of the coating is less than the coefficient of friction between the device and the sheath prior to the application of the coating, and in the range of from about 0.2 to about 0.5.

3. The system of claim 1 or 2, wherein the medical device is a frame and the powder coating is disposed directly on the abluminal surface of the frame.

4. The system of claim 3, wherein the frame is metallic.

5. The system of claim 3 or 4, wherein the frame is a stent.

6. The system of claim 1 or 2, wherein the medical device is covered with a polymeric material and the powder coating is disposed on the polymeric material.

7. The system of claim 6 wherein the polymeric material is a graft material and the device is a stent graft.

8. The system of any preceding claim wherein the luminal surface of the device is coated with the powder coating.

9. A method of manufacturing a medical device system, comprising:
providing a sheath having an abluminal surface and a luminal surface;
providing an expandable device, the device having an abluminal surface and a luminal surface defining a lumen;
applying a coating compound comprising a powder of sodium bicarbonate, sodium maleate, sodium gluconate, sodium fumarate, sodium chloride, magnesium bicarbonate and potassium bicarbonate, on at least one of the abluminal surface of the device and the luminal surface of the sheath, and
disposing the device at least partially within the sheath so that the device is at least partially in contact with the luminal surface of the sheath,
wherein the device and the sheath prior to the application of the coating have at least one of a first property of adhesiveness and a first property of friction when in contact with each other, and subsequent to the application of the coating have at least one of a second property of adhesiveness less than the first property of adhesiveness and a second property of friction less than the first property of friction when in contact with each other.

10. The method of claim 10, wherein the coefficient of friction between the device and the sheath subsequent to the application of the coating is less than the coefficient of friction between the device and the sheath prior to the application of the coating, and in the range of from about 0.2 to about 0.5.

11. The method of claim 10, wherein the step of applying comprises:
dusting at least one of the abluminal surface of the device or the luminal surface of the sheath with a fine powder form of the coating compound;
rolling the abluminal surface of the device over a fine powder coating distributed on a smooth surface;
evaporating an aqueous solution comprising the coating compound from the at least one of the abluminal surface of the device or the luminal surface of the sheath; or
electrospraying a solution comprising the coating compound onto the at least one of the abluminal surface of the device or the luminal surface of the sheath.

12. The method of any of claims 9 to 11, further comprising applying the coating compound to the luminal surface of the device.

13. The method of claim 10, wherein the expandable device is a stent and the method comprises the step of inserting the stent into the sheath in less than 60 minutes.

## Patentansprüche

1. Medizinisches Vorrichtungssystem, umfassend:
eine Hülle mit einer abluminalen Fläche und einer luminalen Fläche;
eine expandierbare medizinische Vorrichtung, die wenigstens teilweise in der Hülle angeordnet ist, wobei die Vorrichtung eine abluminale Fläche, die wenigstens teilweise mit der luminalen Fläche der Hülle in Kontakt ist, und eine luminale Fläche aufweist, die ein Lumen definiert; und
eine Pulverbeschichtung aus einem oder mehreren von Natriumbicarbonat, Natriummaleat, Natriumgluconat, Natriumfumarat, Natriumchlorid, Magnesiumbicarbonat und Kaliumbicarbonat, die wenigstens auf der abluminalen Fläche der Vorrichtung und/oder der luminalen Fläche der Hülle angeordnet ist,
wobei die Vorrichtung und die Hülle vor dem Aufbringen der Beschichtung wenigstens eine erste Haftfestigkeitseigenschaft und/oder eine erste Reibungseigenschaft aufweisen, wenn sie in Kontakt miteinander sind, und nach dem Aufbringen der Beschichtung wenigstens eine zweite Haftfestigkeitseigenschaft, die geringer als die erste Haftfestigkeitseigenschaft ist, und/oder eine zweite Reibungseigenschaft, die geringer als die erste Reibungseigenschaft ist, aufweisen, wenn sie in Kontakt miteinander sind.

2. System nach Anspruch 1, wobei der Reibungskoeffizient zwischen der Vorrichtung und der Hülle nach dem Aufbringen der Beschichtung geringer als der Reibungskoeffizient zwischen der Vorrichtung und der Hülle vor dem Aufbringen der Beschichtung ist und im Bereich von ungefähr 0,2 bis ungefähr 0,5 liegt.

3. System nach Anspruch 1 oder 2, wobei die medizinische Vorrichtung ein Rahmen ist und die Pulverbeschichtung direkt auf der abluminalen Fläche des Rahmens angeordnet ist.

4. System nach Anspruch 3, wobei der Rahmen aus Metall besteht.

5. System nach Anspruch 3 oder 4, wobei der Rahmen ein Stent ist.

6. System nach Anspruch 1 oder 2, wobei die medizinische Vorrichtung mit einem Polymermaterial überzogen ist und die Pulverbeschichtung auf dem Polymermaterial angeordnet ist.

7. System nach Anspruch 6, wobei das Polymermaterial ein Graftmaterial ist und die Vorrichtung ein Stentgraft ist.

8. System nach einem der vorhergehenden Ansprüche, wobei die luminale Fläche der Vorrichtung mit der Pulverbeschichtung beschichtet ist.

9. Verfahren zur Herstellung eines medizinischen Vorrichtungssystems, umfassend:
Bereitstellen einer Hülle mit einer abluminalen Fläche und einer luminalen Fläche;
Bereitstellen einer expandierbaren Vorrichtung, wobei die Vorrichtung eine abluminale Fläche und eine luminale Fläche aufweist, die ein Lumen definiert;
Aufbringen einer Pulverbeschichtung, umfassend ein Pulver aus Natriumbicarbonat, Natriummaleat, Natriumgluconat, Natriumfumarat, Natriumchlorid, Magnesiumbicarbonat und Kaliumbicarbonat, wenigstens auf der abluminalen Fläche der Vorrichtung und/oder der luminalen Fläche der Hülle, und
Anordnen der Vorrichtung wenigstens teilweise in der Hülle, so dass die Vorrichtung wenigstens teilweise mit der luminalen Fläche der Hülle in Kontakt ist,
wobei die Vorrichtung und die Hülle vor dem Aufbringen der Beschichtung wenigstens eine erste Haftfestigkeitseigenschaft und/oder eine erste Reibungseigenschaft aufweisen, wenn sie in Kontakt miteinander sind, und nach dem Aufbringen der Beschichtung wenigstens eine zweite Haftfestigkeitseigenschaft, die geringer als die erste Haftfestigkeitseigenschaft ist, und/oder eine zweite Reibungseigenschaft, die geringer als die erste Reibungseigenschaft ist, aufweisen, wenn sie in Kontakt miteinander sind.

10. Verfahren nach Anspruch 10, wobei der Reibungskoeffizient zwischen der Vorrichtung und der Hülle nach dem Aufbringen der Beschichtung geringer als der Reibungskoeffizient zwischen der Vorrichtung und der Hülle vor dem Aufbringen der Beschichtung ist und im Bereich von ungefähr 0,2 bis ungefähr 0,5 liegt.

11. Verfahren nach Anspruch 10, wobei der Aufbringungsschritt das Folgende umfasst:
Bestäuben wenigstens der abluminalen Fläche der Vorrichtung und/oder der luminalen Fläche der Hülle mit einer feinen Pulverform der Beschichtungsverbindung;
Rollen der abluminalen Fläche der Vorrichtung über eine feine Pulverbeschichtung, die auf einer glatten Fläche verteilt ist;
Verdunsten einer wässrigen Lösung, welche die Beschichtungsverbindung umfasst, von wenigstens der abluminalen Fläche der Vorrichtung und/oder der luminalen Fläche der Hülle; oder
Elektrosprayen einer Lösung, welche die Beschichtungsverbindung umfasst, wenigstens auf die abluminale Fläche der Vorrichtung und/oder die luminale Fläche der Hülle.

12. Verfahren nach einem der Ansprüche 9 bis 11, ferner umfassend Aufbringen der Beschichtungsverbindung auf die luminale Fläche der Vorrichtung.

13. Verfahren nach Anspruch 10, wobei die expandierbare Vorrichtung ein Stent ist und das Verfahren den Schritt der Einführung des Stents in die Hülle in weniger als 60 Minuten umfasst.

## Revendications

1. Système de dispositif médical comprenant :
une gaine présentant une surface abluminale et une surface luminale ;
un dispositif médical extensible disposé au moins partiellement à l'intérieur de la gaine, le dispositif comportant une surface abluminale, au moins partiellement en contact avec la surface luminale de la gaine, et une surface luminale délimitant une lumière ; et
un poudrage au moyen d'un ou plusieurs produits parmi bicarbonate de sodium, maléate de sodium, gluconate de sodium, fumarate de sodium, chlorure de sodium, bicarbonate de magnésium et bicarbonate de potassium, disposés sur la surface abluminale du dispositif et/ou la surface luminale de la gaine,
dans lequel le dispositif et la gaine, avant l'application du revêtement, ont une première propriété d'adhésivité et/ou une première propriété de frottement quand ils sont en contact l'un avec l'autre et, après l'application du revêtement, ont une seconde propriété d'adhésivité inférieure à la première propriété d'adhésivité et/ou une seconde propriété de frottement inférieure à la première propriété de frottement quand ils sont en contact l'un avec l'autre.

2. Système selon la revendication 1, dans lequel le coefficient de frottement entre le dispositif et la gaine après l'application du revêtement est inférieur au coefficient de frottement entre le dispositif et la gaine avant l'application du revêtement et se situe dans la plage allant d'environ 0,2 à environ 0,5.

3. Système selon la revendication 1 ou 2, dans lequel le dispositif médical est une armature et le revêtement en poudre est disposé directement sur la surface abluminale de l'armature.

4. Système selon la revendication 3, dans lequel l'armature est métallique.

5. Système selon la revendication 3 ou 4, dans lequel l'armature est une endoprothèse vasculaire.

6. Système selon la revendication 1 ou 2, dans lequel le dispositif médical est recouvert d'une matière polymère et le revêtement en poudre est disposé sur la matière polymère.

7. Système selon la revendication 6, dans lequel la matière polymère est un polymère greffé et le dispositif est une greffe d'endoprothèse vasculaire.

8. Système selon l'une quelconque des revendications précédentes, dans lequel la surface luminale du dispositif est revêtue avec le revêtement en poudre.

9. Procédé de fabrication d'un système de dispositif médical comprenant les étapes consistant à:
produire une gaine présentant une surface abluminale et une surface luminale ;
produire un dispositif médical extensible, le dispositif comportant une surface abluminale et une surface luminale délimitant une lumière ;
appliquer un composé de revêtement comprenant une poudre de bicarbonate de sodium, maléate de sodium, gluconate de sodium, fumarate de sodium, chlorure de sodium, bicarbonate de magnésium et bicarbonate de potassium sur la surface abluminale du dispositif et/ou la surface luminale de la gaine, et
mettre en place le dispositif au moins partiellement à l'intérieur de la gaine de sorte que le dispositif soit au moins partiellement en contact avec la surface luminale de la gaine,
dans lequel le dispositif et la gaine, avant l'application du revêtement, ont une première propriété d'adhésivité et/ou une première propriété de frottement quand ils sont en contact l'un avec l'autre et, après l'application du revêtement, ont une seconde propriété d'adhésivité inférieure à la première propriété d'adhésivité et/ou une seconde propriété de frottement inférieure à la première propriété de frottement quand ils sont en contact l'un avec l'autre.

10. Procédé selon la revendication 10, dans lequel le coefficient de frottement entre le dispositif et la gaine après l'application du revêtement est inférieur au coefficient de frottement entre le dispositif et la gaine avant l'application du revêtement et se situe dans la plage allant d'environ 0,2 à environ 0,5.

11. Procédé selon la revendication 10, dans lequel l'étape d'application comprend les opérations consistant à :
poudrer la surface abluminale du dispositif et/ou la surface luminale de la gaine avec une forme pulvérulente fine du composé de revêtement ;
rouler la surface abluminale du dispositif sur un revêtement en poudre fine réparti sur une surface lisse ;
évaporer une solution aqueuse comprenant le composé de revêtement de la surface abluminale du dispositif et/ou de la surface luminale de la gaine, ou
électronébuliser une solution comprenant le composé de revêtement sur la surface abluminale du dispositif et/ou sur la surface luminale de la gaine.

12. Procédé selon l'une quelconque des revendications 9 à 11, comprenant en outre l'application du composé de revêtement sur la surface luminale du dispositif.

13. Procédé selon la revendication 10, dans lequel le dispositif extensible est une endoprothèse vasculaire et le procédé comprend l'étape d'introduction de l'endoprothèse vasculaire dans la gaine en moins de 60 minutes.
